(19) Europäisches Patentamt / European Patent Office / Office européen des brevets

(11) **EP 4 414 699 B1**

(12) **EUROPEAN PATENT SPECIFICATION**

(45) Date of publication and mention of the grant of the patent:
**24.09.2025 Bulletin 2025/39**

(21) Application number: **23156358.6**

(22) Date of filing: **13.02.2023**

(51) International Patent Classification (IPC):
**G01N 27/12** (2006.01)    **G01N 33/00** (2006.01)

(52) Cooperative Patent Classification (CPC):
**G01N 33/0006; G01N 27/122; G01N 33/0034**

(54) **GAS SENSING DEVICE AND METHOD FOR OPERATING A GAS SENSING DEVICE**

GASSENSORVORRICHTUNG UND VERFAHREN ZUM BETREIBEN EINER GASSENSORVORRICHTUNG

DISPOSITIF DE DÉTECTION DE GAZ ET PROCÉDÉ DE FONCTIONNEMENT D'UN DISPOSITIF DE DÉTECTION DE GAZ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**14.08.2024 Bulletin 2024/33**

(73) Proprietor: **Infineon Technologies AG**
**85579 Neubiberg (DE)**

(72) Inventors:
• **ITEN, Luca**
  **6023 Rothenburg (CH)**
• **CARBONELLI, Cecilia**
  **81477 München (DE)**
• **ZHAO, Jianyu**
  **81539 München (DE)**

• **MITTERMAIER, Simon**
  **85659 Forstern (DE)**

(74) Representative: **Hersina, Günter et al**
**Schoppe, Zimmermann, Stöckeler**
**Zinkler, Schenk & Partner mbB**
**Patentanwälte**
**Radlkoferstrasse 2**
**81373 München (DE)**

(56) References cited:
**EP-A1- 3 936 861    CN-A- 112 418 395**

• **FENG SHAOBIN ET AL: "Review on Smart Gas Sensing Technology", SENSORS, vol. 19, no. 17, 30 August 2019 (2019-08-30), pages 3760, XP055953601, DOI: 10.3390/s19173760**

**Description**

Technical Field

The present invention relates to a gas sensing device for sensing one or more gases in a mixture of gases and to a method for operating such gas sensing device. More particular, the disclosure deals with the estimation of gas concentrations through the use of chemo-resistive gas sensors.

Background of the Invention

Chemo-resistive gas sensors change their electrical properties in the presence of gases such as nitrogen dioxide (NO2), ozone (O3), ammonia (NH3) or carbon monoxide (CO) so that a concentration of such gases in a mixture of gases may be determined by measuring the electrical properties of one or more chemo-resistive gas sensors. However, chemo-resistive gas sensors are prone to the effect of drifting, which is the effect that the sensor outputs change (i.e. drift) over time for similar gas concentrations. This makes the estimation of the gas concentrations harder the longer the chemo-resistive gas sensors are in operation up to the point where it, unfortunately, fails completely. The drift behavior of gas sensors in general is a well-known and documented problem.

EP3936861 discloses a gas sensor, a preprocessing and drift-correcting processor, a feature extractor and a sensing result processor comprising a trained model. A processor comprising another trained model can calculate a new baseline, thereby updating a drift correction.

Summary of the Invention

A gas sensing device for sensing one or more gases in a mixture of gases is provided. The gas sensing device comprises:

one or more chemo-resistive gas sensors, wherein each of the gas sensors is configured for generating signal samples corresponding to concentrations of the one or more gases in the mixture of gases;

a preprocessing processor configured for preprocessing the signal samples in order to generate a preprocessed signal sample for each of the signal samples;

a drift correction processor configured for applying a mapping function to each of the preprocessed signal samples in order to create a drift-corrected signal sample for each of the preprocessed signal samples, wherein the drift correction processor is configured for receiving updates for the mapping function, wherein each update initiates an update step of a plurality of consecutive update steps;

a feature extraction processor configured for extracting a set of feature values from each of the drift-corrected signal samples based on characteristics of the received drift-corrected signal samples;

a sensing result processor configured for creating for each of the gases a sensing result for each of the sets of feature values, wherein the sensing result processor comprises a first trained model based algorithm processor and a first trained model for the first trained model based algorithm processor, wherein the sets of feature values are fed to an input side of the first trained model based algorithm processor, and wherein the sensing results are provided at an output side of the first trained model based algorithm processor;

a state processor configured for determining a state of the gas sensing device for a current update step of the update steps, wherein the state comprises at least a part of the preprocessed signal samples of the current update step, at least a part of the preprocessed signal samples of previous update steps of the update steps and the mapping function of the current update step; and

an update processor configured for providing the update for a subsequent update step subsequently following the current update step to the drift correction processor, wherein the update processor comprises a second trained model based algorithm processor, a second trained model for the second trained model based algorithm processor and an update action processor, wherein the state of the current update step is fed to an input side of the second trained model based algorithm processor, wherein a quality value for each possible update action of a plurality of update actions is provided at an output side of the second trained model based algorithm processor, wherein the update action processor is configured for calculating the update for the subsequent update step by applying the possible update action, which has a highest quality value of the quality values of the current update step, to the mapping function of the current update step.

The one or more chemo-resistive gas sensors may be reduced graphene gas sensors, where the base material is functionalized with specific chemicals, e.g. with platinum (Pt), or manganese dioxide (MnO2), so that each of the gas sensors is sensitive for a specific gas, e.g. for nitrogen dioxide (NO2), ozone (O3), ammonia (NH3) or carbon monoxide (CO). In doing so, the interaction between graphene sheets and absorbed gas analytes influences the electronic structure of the material depending on the mixture of gases, resulting in altered charge carrier concentration and changed electrical conductance.

In case of multi-gas sensing a multi-gas sensor array comprising a plurality of chemo-resistive gas sensors having dissimilar selectivity may be used. Due to the different sensitivity towards various gas molecules, resistances of the gas sensors change in disparate patterns, making it possible to analyze complicated gas mixtures with one single sensor array.

A signal sample is a sequence consisting of time-discrete signal values, wherein the signal values are output by one of the gas sensors.

The term processor refers to an electronic device configured for specific task. A processor may comprise hardware or a combination of hardware and software. Different processors may share hardware components and/or software components.

The pre-processing processor is configured for suppressing and/or compensating of artifacts in the signal samples and/or noise in the signal samples and/or invalid signal samples due to malfunctioning gas sensors in order to produce more reliable preprocessed signal samples.

The drift correction processor is configured for applying a mapping function to each of the preprocessed signal samples in order to create a drift-corrected signal sample for each of the preprocessed signal samples during an operational phase. The drift correction processor is configured for receiving updates for the mapping function during the operation phase, wherein each update initiates an update step of a plurality of consecutive update steps. A new update step, in which a new update for the mapping function is provided, may be initiated, for example, every 60 seconds.

The feature extraction processor is configured for receiving the drift-corrected signal samples and for extracting a set of feature values from each of the received drift-corrected signal samples of each of the gas sensors based on characteristics of the received drift-corrected signal samples of the respective gas sensor. The features may be based on dynamic characteristics of the signal samples.

A trained model based algorithm processor is a processor, which is capable of machine learning. The machine learning is done in a preoperational training phase in which trained models are developed by comparing actual output values of the trained model based algorithm stage with desired output values of the trained model based algorithm stage for defined inputs of the trained model based algorithm stage. The trained models have a predefined structure, wherein a parametrization of the predefined structure is done during the training phase. The trained models comprise the learned content after the training phase is finished. In an operational phase for producing processing results one or more of the trained models from the training phase are used to process their input data.

In the training phase, the plurality of trained models can be established and afterwards stored at the gas sensing device. The trained models may differ in the structures and/or the parameters. During the operation phase the most appropriate trained model may be selected depending on the specific use-case.

The state processor is configured for determining a state of the gas sensing device for a current update step of the update steps, wherein the state comprises at least a part of the preprocessed signal samples of the current update step, at least a part of the preprocessed signal samples of previous update steps of the update steps and the mapping function of the current update step. For example, the state processor may use every tenth preprocessed signal sample of the previous 200 update steps.

The update processor is configured for providing the update for a subsequent update step subsequently following the current update step to the drift correction processor, wherein the update processor comprises a second trained model based algorithm processor, a second trained model for the second trained model based algorithm processor and an update action processor, wherein the state of the current update step is fed to an input side of the second trained model based algorithm processor, wherein a quality value for each possible update action of a plurality of update actions is provided at an output side of the second trained model based algorithm processor, wherein the update action processor is configured for calculating the update for the subsequent update step by applying the possible update action, which has a highest quality value of the quality values of the current update step, to the mapping function of the current update step.

The disclosure provides a new approach to tackle the drift problem of chemo-resistive gas sensors using a drift correction processor for applying a mapping function to the preprocessed signal samples and a second trained model based algorithm processor for providing updates for the mapping function. The core of the idea is the presence of a second trained model based algorithm processor, which explores how different update for the mapping function applied to the preprocessed signal samples affect the performance of the predictions and eventually selects the best update for the mapping function for the specific drift experienced in the field without any a-priori knowledge of the drift rate.

The approach can be elegantly overlapped to an existing signal processing and algorithm flows with the goal of stabilizing the drift and improving the final gas predictions. In other words, calibration models trained for un-drifted data can be made usable for drifted data as well in real-world conditions.

The gas sensing device according to the disclosure provides a better long-term stability of the sensing results than approaches which seek to solve the drifting problem by training the first trained model of the first trained model based algorithm processor of the sensing result processor with drifted data as such data do usually not cover all possible drifting scenarios. Even if that would not be a problem, training the sensing result processor in the field on such a great amount of data would exceed the possibilities of the used processing units.

Furthermore, the gas sensing device according to the disclosure provides a better long-term stability of the sensing results than approaches which seek to solve the drifting problem by regularly updating the baseline, which normalizes the sensor outputs, as this baseline has to be calculated with sensor exposure to 0 ppb target gas periodically, which again implies restrictions on the applicability of the algorithms.

According to embodiments of the disclosure, the update processor is configured to detect a change of a drift behavior of one or more of the one or more chemo-resistive gas sensors, which can not be compensated by the updates. The change of the drift behavior, which can not be compensated by the updates may be detected by analyzing the quality values for each of the possible update actions. For example, a change in the drift behavior may be assumed if the highest quality factor is smaller than a preset value.

According to embodiments of the disclosure, the update processor is configured to initiate a training procedure for training the second trained model in case that the change of the drift behavior, which can not be compensated by the updates, is detected. By such features, the long-term stability of the sensing results may be further increased.

According to embodiments of the disclosure, the second trained model based algorithm processor is a reinforcement learning processor and the second trained model is a reinforcement learning model, wherein the gas sensing device comprises a reward processor configured for calculating a reward, which is fed to an input side of the reinforcement learning processor during the training procedure, in order to train the reinforcement learning model. Some applications of known reinforcement learning principles in different fields are described in References [1] and [2]. However, it is possible to use other machine- learning approaches for the second trained model based algorithm processor

According to embodiments of the disclosure, the reward processor is configured for calculating the reward depending on the sensor results created by the sensing result processor during the training procedure and depending on reference sensing results provided by an external source for the training procedure. By such features, the long-term stability of the sensing results may be further increased.

According to embodiments of the disclosure, the reward processor is configured for calculating the reward depending on the sets of feature values created by the feature extraction processor during the training procedure by analyzing a first subset of the feature values of each set of feature values and by analyzing a second subset of the feature values of each set of feature values, wherein the feature values of the first subset are more drift-dependent than the feature values of the second subset.

By such features, the long-term stability of the sensing results may be further increased. Moreover, no reference sensing results are needed.

According to embodiments of the disclosure, the first trained model based algorithm processor is implemented as a gated recurrent unit regressor. A gated recurrent regressor is recurrent neural network using gating mechanisms.

According to embodiments of the disclosure, the second trained model based algorithm processor is implemented as a deep neural network. A deep neural network is an artificial neural network with multiple layers between the input and output layers.

Further disclosed is a method for operating a gas sensing device for sensing one or more gases in a mixture of gases, wherein the gas sensing device comprises one or more chemo-resistive gas sensors, wherein the method comprises the steps of:

> using each of the gas sensors for generating signal samples corresponding to concentrations of the one or more gases in the mixture of gases;

> using a preprocessing processor of the gas sensing device for preprocessing the signal samples in order to generate a preprocessed signal sample for each of the signal samples;

> using a drift correction processor of the gas sensing device for applying a mapping function to each of the preprocessed signal samples in order to create a drift-corrected signal sample for each of the preprocessed signal samples, wherein the drift correction processor is configured for receiving updates for the mapping function, wherein each update initiates an update step of a plurality of consecutive update steps;

using a feature extraction processor of the gas sensing device for extracting a set of feature values from each of the drift-corrected signal samples based on characteristics of the received drift-corrected signal samples;

using a sensing result processor of the gas sensing device for creating for each of the gases a sensing result for each of the sets of feature values, wherein the sensing result processor comprises a first trained model based algorithm processor and a first trained model for the first trained model based algorithm processor, wherein the sets of feature values are fed to an input side of the first trained model based algorithm processor, and wherein the sensing results are provided at an output side of the first trained model based algorithm processor;

using a state processor of the gas sensing device for determining a state of the gas sensing device for a current update step of the update steps, wherein the state comprises at least a part of the preprocessed signal samples of the current update step, at least a part of the preprocessed signal samples of previous update steps of the update steps and the mapping function of the current update step; and

using an update processor of the gas sensing device for providing the update for a subsequent update step subsequently following the current update step to the drift correction processor, wherein the update processor comprises a second trained model based algorithm processor, a second trained model for the second trained model based algorithm processor and an update action processor, wherein the state of the current update step is fed to an input side of the second trained model based algorithm processor, wherein a quality value for each possible update action of a plurality of update actions is provided at an output side of the second trained model based algorithm processor, wherein the update action processor is configured for calculating the update for the subsequent update step by applying the possible update action, which has a highest quality value of the quality values of the current update step, to the mapping function of the current update step.

Brief Description of the Drawings

Preferred embodiments of the invention are subsequently discussed with respect to the accompanying drawings, in which:

Figure 1    shows a schematic view of a first exemplary embodiment of a gas sensing device according to the disclosure;

Figure 2    shows a schematic view of a second exemplary embodiment of a gas sensing device according to the disclosure;

Figure 3    shows a schematic view of a third exemplary embodiment of a gas sensing device according to the disclosure;

Figure 4    illustrates an exemplary method for operating the second or third exemplary embodiment of a gas sensing device according to the disclosure;

Figure 5    illustrates exemplary sensing results over time of a gas sensing device according to the disclosure and exemplary prior art sensing results over time of a gas sensing device according to prior art compared to reference sensing results;

Figure 6    illustrates an exemplary features ratio between a non-drifting feature value of the sets of feature values and a drifting feature value of the sets of feature values over time for different time intervals;

Figure 7    illustrates exemplary sensing results of a gas sensing device according to the disclosure and exemplary prior art sensing results of a gas sensing device according to prior art compared to reference sensing results for nitrogen dioxide and ozone as well as the associated reward and the associated prior art reward;

Figure 8    illustrates an exemplary accumulated reward for different correction factors over time; and

Figure 9    illustrates an exemplary accumulated reward for different depreciation factors over time.

Equal or equivalent elements or elements with equal or equivalent functionality are denoted in the following description by equal or equivalent reference numerals.

In the following description, a plurality of details is set forth to provide a more thorough explanation of embodiments of the present disclosure. However, it will be apparent to those skilled in the art that embodiments of the present disclosure may be practiced without these specific details. In other instances, well-known structures and devices are shown in block diagram form rather than in detail in order to avoid obscuring embodiments of the present disclosure. In addition, features of the different embodiments described hereinafter may be combined with each other, unless specifically noted otherwise.

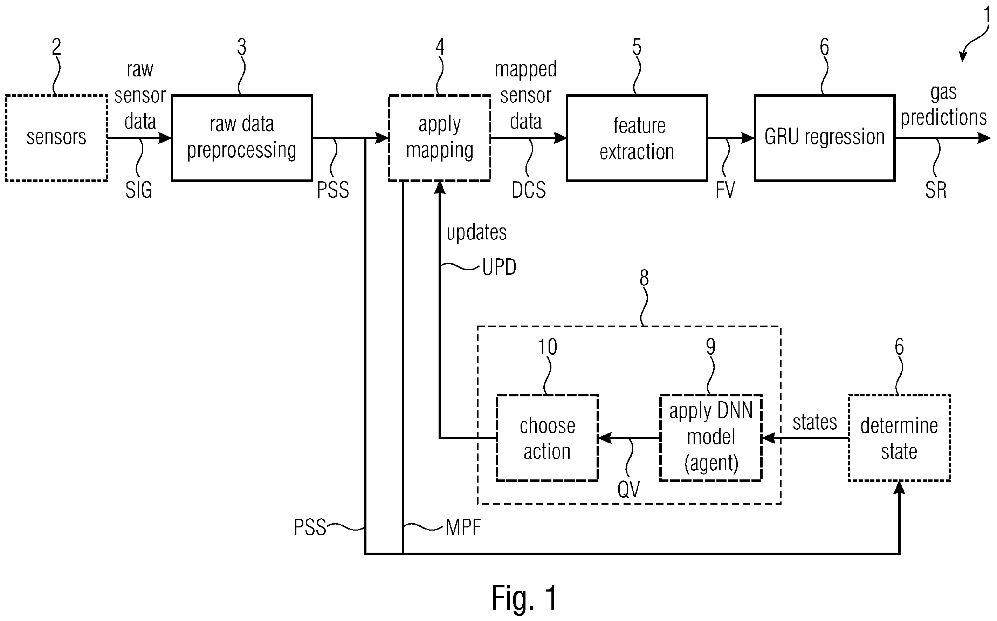

Figure 1 shows a schematic view of a first exemplary embodiment of a gas sensing device 1 according to the disclosure.

A gas sensing device for sensing one or more gases in a mixture of gases. The gas sensing device 1 comprises:

one or more chemo-resistive gas sensors 2, wherein each of the gas sensors 2 is configured for generating signal samples SIG corresponding to concentrations of the one or more gases in the mixture of gases;

a preprocessing processor 3 configured for preprocessing the signal samples SIG in order to generate a preprocessed signal sample PSS for each of the signal samples SIG;

a drift correction processor 4 configured for applying a mapping function MPF to each of the preprocessed signal samples PSS in order to create a drift-corrected signal sample DCS for each of the preprocessed signal samples PSS, wherein the drift correction processor 4 is configured for receiving updates UPD for the mapping function MPF, wherein each update UPD initiates an update step of a plurality of consecutive update steps;

a feature extraction processor 5 configured for extracting a set of feature values FV from each of the drift-corrected signal samples DCS based on characteristics of the received drift-corrected signal samples DCS;

a sensing result processor 6 configured for creating for each of the gases a sensing result SR for each of the sets of feature values FV, wherein the sensing result processor 6 comprises a first trained model based algorithm processor and a first trained model for the first trained model based algorithm processor, wherein the sets of feature values FV are fed to an input side of the first trained model based algorithm processor, and wherein the sensing results SR are provided at an output side of the first trained model based algorithm processor;

a state processor 7 configured for determining a state STA of the gas sensing device 1 for a current update step of the update steps, wherein the state STA comprises at least a part of the preprocessed signal samples PSS of the current update step, at least a part of the preprocessed signal samples PSS of previous update steps of the update steps and the mapping function MPF of the current update step; and

an update processor 8 configured for providing the update UPD for a subsequent update step subsequently following the current update step to the drift correction processor 4, wherein the update processor 8 comprises a second trained model based algorithm processor 9, a second trained model for the second trained model based algorithm processor 9 and an update action processor 10, wherein the state STA of the current update step is fed to an input side of the second trained model based algorithm processor 9, wherein a quality value QV for each possible update action of a plurality of update actions is provided at an output side of the second trained model based algorithm processor 9, wherein the update action processor 10 is configured for calculating the update UPD for the subsequent update step by applying the possible update action, which has a highest quality value QV of the quality values QV of the current update step, to the mapping function MPF of the current update step.

According to embodiments of the disclosure, the first trained model based algorithm processor is implemented as a gated recurrent unit regressor.

According to embodiments of the disclosure, the second trained model based algorithm processor 9 is implemented as a deep neural network.

In prior art approaches, the signal samples SIG of the chemo-resistive sensors 2 are received and preprocessed by the preprocessing processor 3. Then, the needed sets of feature values FV are directly extracted by the feature extraction processor 5 from the preprocessed signal samples PSS. In a last step, these sets of feature values FV are feed to a first trained model based algorithm processor 6, which may be a gated recurrent unit regression network doing the sensing results SR. These sensing results SR, however, are heavily influenced by drift.

In contrast to that, the proposed approach uses an additional machine-learning algorithm, in particular a reinforcement learning algorithm, to "learn" an adaptive mapping for the preprocessed signal samples PSS. Said mapping can then be used to convert the preprocessed signal samples PSS into drift-corrected signal samples DCS, ideally correcting the occurring drift over time. Sensing results SR derived from sets of feature values FV based on these drift-corrected signal samples DCS are now supposed to be more accurate than the sensing results SR derived from sets of feature values FV based on the unmapped preprocessed signal samples PSS, i.e. the proposed solution improves the sensing results SR for chemo-resistive sensors 2 that drift over time.

Figure 1 shows the processing flow of the gas sensing device 1 during an operational phase under the assumption that the first trained model of the sensing result processor 6 and the second trained model of the update processor 8 are fully trained.

In a further aspect, the disclosure refers to a method for operating gas a sensing device 1 for sensing one or more gases in a mixture of gases, wherein the gas sensing device 1 comprises one or more chemo-resistive gas sensors 2, wherein the method comprises the steps of:

using each of the gas sensors 2 for generating signal samples SIG corresponding to concentrations of the one or more gases in the mixture of gases;

using a preprocessing processor 3 of the gas sensing device 1 for preprocessing the signal samples SIG in order to generate a preprocessed signal sample PSS for each of the signal samples SIG;

using a drift correction processor 4 of the gas sensing device 1 for applying a mapping function MPF to each of the preprocessed signal samples PSS in order to create a drift-corrected signal sample DCS for each of the preprocessed signal samples PSS, wherein the drift correction processor 4 is configured for receiving updates UPD for the mapping function MPF, wherein each update UPD initiates an update step of a plurality of consecutive update steps;

using a feature extraction processor 5 of the gas sensing device 1 for extracting a set of feature values FV from each of the drift-corrected signal samples DCS based on characteristics of the received drift-corrected signal samples DCS;

using a sensing result processor 6 of the gas sensing device 1 for creating for each of the gases a sensing result SR for each of the sets of feature values FV, wherein the sensing result processor 6 comprises a first trained model based algorithm processor and a first trained model for the first trained model based algorithm processor, wherein the sets of feature values FV are fed to an input side of the first trained model based algorithm processor, and wherein the sensing results SR are provided at an output side of the first trained model based algorithm processor;

using a state processor 7 of the gas sensing device 1 for determining a state STA of the gas sensing device 1 for a current update step of the update steps, wherein the state STA comprises at least a part of the preprocessed signal samples PSS of the current update step, at least a part of the preprocessed signal samples PSS of previous update steps of the update steps and the mapping function MPF of the current update step; and

using an update processor 8 of the gas sensing device 1 for providing the update UPD for a subsequent update step subsequently following the current update step to the drift correction processor 4, wherein the update processor 8 comprises a second trained model based algorithm processor 9, a second trained model for the second trained model based algorithm processor 9 and an update action processor 10, wherein the state STA of the current update step is fed to an input side of the second trained model based algorithm processor 9, wherein a quality value QV for each possible update action of a plurality of update actions is provided at an output side of the second trained model based algorithm processor 9, wherein the update action processor 10 is configured for calculating the update UPD for the subsequent update step by applying the possible update action, which has a highest quality value QV of the quality values of the current update step, to the mapping function MPF of the current update step.

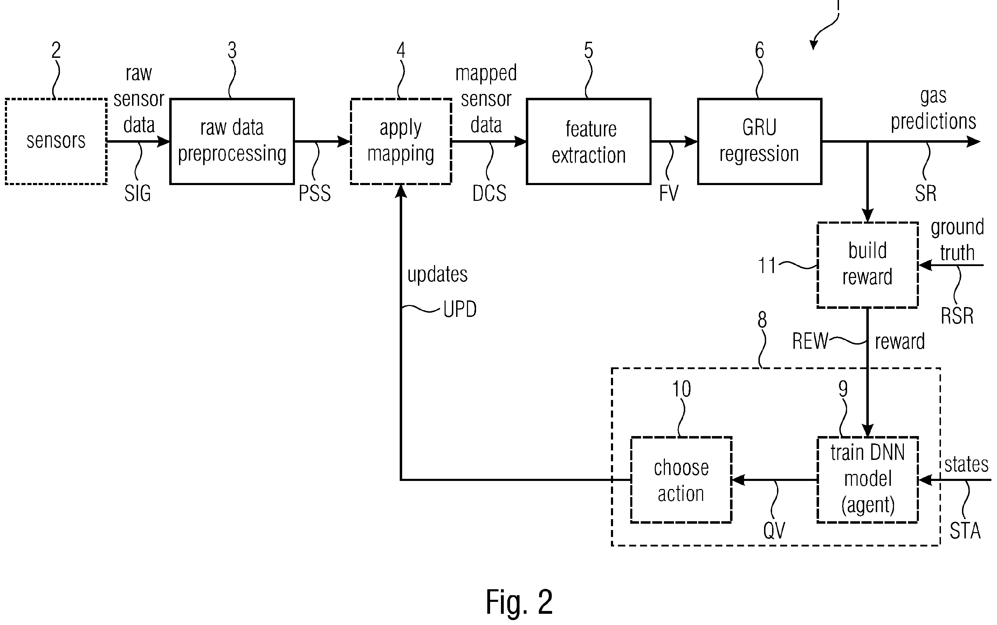

Figure 2 shows a schematic view of a second exemplary embodiment of a gas sensing device 1 according to the disclosure.

According to embodiments of the disclosure, the second trained model based algorithm processor 9 is a reinforcement learning processor and the second trained model is a reinforcement learning model, wherein the gas sensing device 1 comprises a reward processor 11 configured for calculating a reward REW, which is fed to an input side of the reinforcement learning processor 9 during the training procedure TP, in order to train the reinforcement learning model.

According to embodiments of the disclosure, the reward processor 11 is configured for calculating the reward REW depending on the sensor results SR created by the sensing result processor 6 during the training procedure TP and depending on reference sensing results RSR provided by an external source for the training procedure TP.

Figure 2 shows the processing flow of the gas sensing device 1 during an initial training phase TP when the second trained model based algorithm processor 9 is still not trained or during a subsequent training phase TP when the second trained model based algorithm processor 9 has to be trained again because the drift has significantly changed (e.g. it has become more severe, for example, from linear to exponential), wherein the reward REW is calculated from the sensor results SR created by the sensing result processor 6 during the training procedure TP and depending on reference sensing results RSR provided by an external source for the training procedure TP.

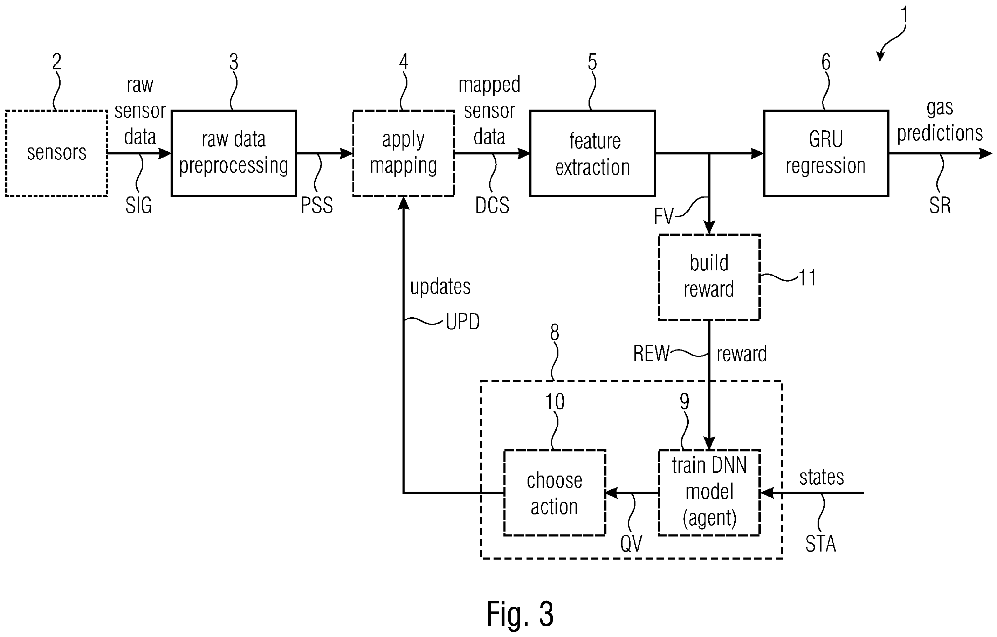

Figure 3 shows a schematic view of a third exemplary embodiment of a gas sensing device 1 according to the disclosure.

According to embodiments of the disclosure, the reward processor 11 is configured for calculating the reward REW depending on the sets of feature values FV created by the feature extraction processor 5 during the TP procedure by analyzing a first subset of the feature values FV of each set of feature values FV and by analyzing a second subset of the feature values FV of each set of feature values FV, wherein the feature values FV of the first subset are more drift-dependent than the feature values FV of the second subset.

Figure 3 shows the processing flow of the gas sensing device 1 during an initial training phase TP when the second trained model based algorithm processor 9 is still not trained or during a subsequent training phase TP when the second trained model based algorithm processor 9 has to be trained again because the drift has significantly changed (e.g. it has become more severe, for example, from linear to exponential), wherein the reward REW is calculated from the sets of feature values FV created by the feature extraction processor 5 during the training procedure TP by analyzing a first subset of the feature values FV of each set of feature values FV and by analyzing a second subset of the feature values FV of each set of feature values FV, wherein the feature values FV of the first subset are more drift-dependent than the feature values FV of the second subset.

This section describes how the general invention described in Figures 1-3 may be implemented in chronological order, i.e. in the order the algorithm executes the different parts per update step. A new update step can be performed each time enough new preprocessed signal samples PSS have been observed to construct a new set of feature values (done in the pre-processing processor 3/ feature extraction processor 5), e.g. one update step every 60 seconds. Furthermore, it shall be noted that the following described approach implements the idea of deep Q-learning (DQN), but could have also been done implementing other machine learning algorithms. Some known gradient descent methods (like the Actor-Critic approach) could be also suited.

At the beginning of each update step the current state STA must be constructed / observed by the state processor 7. In the described implementation, the states STA are based on preprocessed signal samples PSS observed in the current update step and on information gathered during a selection of previous update steps (number of steps and their indices relative to the current one can be configured). Furthermore, the current mapping function MPF needs to be included in the state STA, enabling the second trained model based algorithm processor 9 (the agent) to evaluate how the current mapping function MPF affects the observed preprocessed signal samples PSS and to determine how it should be improved.

One common configuration choice is to base the state STA on preprocessed signal samples PSS observed in the current and every tenth outputs from the previous 200 update steps (previously observed preprocessed signal samples PSS stored in a memory). This data is then mapped according to the current mapping, yielding drift-corrected signal samples DCS from the corresponding update steps as they would have been observed with the current mapping (which has probably changed over the past 200 steps).

Now that the state processor 7 knows the current state STA (from which it is possible to infer the drift), this state STA is feed to the second trained model based algorithm processor (9), which may be a deep neural network (in deep Q-learning called "model") estimating the quality values QV for all possible update actions. Possible update actions are changes to the mapping function MPF like increase or decrease one of the mapping parameters (explained later) by an amount (correction factor) that can be configured or doing nothing. It is important to understand that they do not directly affect the preprocessed signal samples PSS. For example, the update action "do nothing" means the mapping function won't be changed in this update step, but it is still applied to the preprocessed signal samples PSS. In deep Q-learning, the update action with the highest estimated quality value QV is chosen with probability p=1- ε and a random action with probability p=ε (called ε-greedy policy). In this implementation ε may be decreasing over time (common for deep-Q-learning).

The number of layers and units per layer for the used deep neural network can be configured. A common choice would be to use 4 dense layers and 1000 units per layer. The used optimizer is Adam with a learning rate decreasing over time. This decrease may depend on how much the quality value estimate error decreased over the past episodes, wherein for example between 2 and 10 episodes are considered.

Next, the (probably altered) mapping function MPF is applied to the preprocessed signal samples PSS. This mapping may be implemented as a vector (array) saving the underlying mapping parameters of a function approximating the drift. Two such functions have been implemented. They make explicit assumptions about the drift behavior. They may either assume it to be linear or exponential and simply additional to the "true" sensor outputs. The parameters a_0 and a_1 are the mentioned mapping parameters (unknown) and t corresponds to the time:

$$drift_{linear} = a_0 + t * a_1$$

$$drift_{exponential} = \exp(a_0 + t * a_1)$$

The drift can either be assumed to be the same or different for each of the chemo-resistive gas sensors 2, resulting in the following number of different actions, where factor 4 is due to the two parameters of which each can be increased or decreased and +1 for the action "do nothing":

$$\#actions = 4 * \#sensors + 1$$

$$\#actions = 4 * 1 + 1 = 4$$

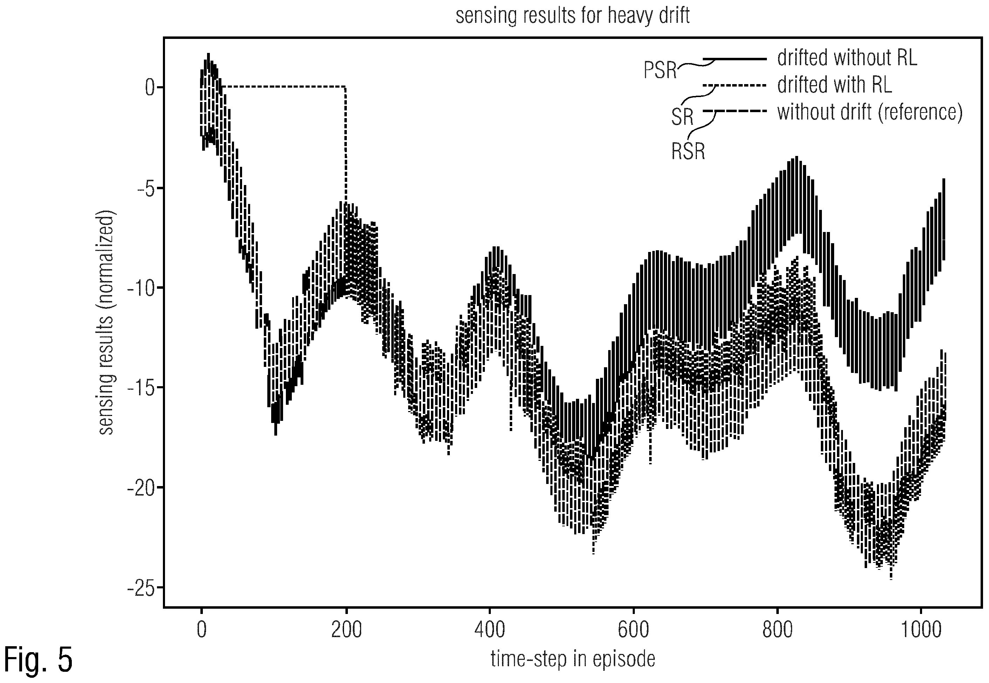

The time t in the equations above correspond to the moment the considered preprocessed signal samples PSS have been observed / measured. An example of how the preprocessed signal samples PSS are mapped is shown in Figure 5.

As shown in Figure 2, the sensing results SR may be leveraged relative to the reference sensing results RSR (true gas concentrations, ground truth) for the reward REW. Different options for the reward function have been implemented. The best performing choice turned out to be the negative Euclidean distance between the sensing results SR and the reference sensing results RSR. This can be formulated in the following way, where $\vec{y} \in R^2$ is the sensing result SR $\vec{\hat{y}} \in R^2$ the reference sensing result, $r_{gt}$ the reward REW, and $\|.\|$ the Euclidean distance (i.e. $l^2$ norm):

$$r_{gt} = - \left\| \vec{y} - \vec{\hat{y}} \right\| \le 0, \qquad r_{gt} \in R^1$$

An example of how the reward REW over one episode can look like is shown in Fig-ure 7.

The disclosed approach can be - as shown in Figure 3 - adapted to the case where the reference sensing results RSR are not available by leveraging sets of feature values FV directly extracted from the drift-corrected signal samples DCS and which are differently affected from drift. By monitoring the relationship between such signals over time, a reward REW can be judiciously built, for example, by combining drift dependent feature values FV (e.g. time features), $F_d(t)$, and features values FV immune to baseline drift, $F_{nd}(t)$ (e.g. frequency dependent features) as described below.

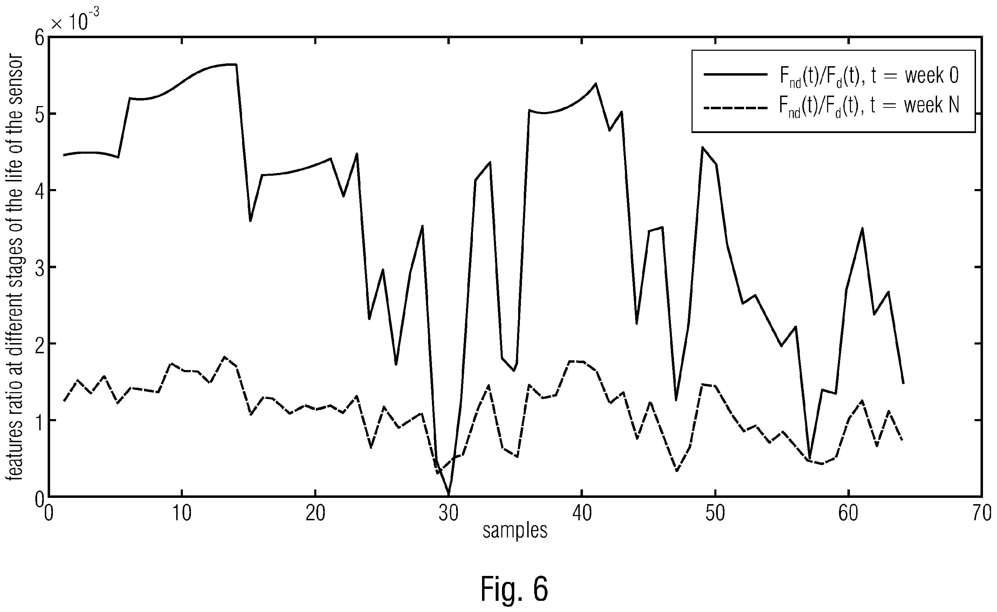

The ratio of drift independent and drift dependent features may be monitored at the lower gas concentrations $C_{gas\_min}$ (or other reference value which occurs periodically) as illustrated in Figure 6 where after a few weeks (5 in the experiments) the ratio has considerably decreased (due to the drift of the sensitivity feature values FV versus the total harmonic distortion).

$$r_f = -\Delta\left(t, C_{gas\_min}\right) = - \left\| \frac{F_{nd}(t)}{F_d(t)} - \frac{F_{nd}(t=0)}{F_d(t=0)} \right\| , \quad r_f \in R^1$$

If $\Delta(t, C_{gas\_min})$ increases, this is a sign of drift that needs to be correct and vice versa.

Alternatively, the correlation between drifting and non-drifting feature values FV can be obtained and a polynomial equation extracted for a given time interval. The parameter of this polynomial fit can be monitored over time and used to build the reward REW.

It should be noted that the resistance of a heater of the chemo-resistive gas sensor(s) 2 could also be used as 'non-drifting' feature value FV (or alternative reference signal available at the sensor 2, which does not vary with the gas concentration).

For training, as common in DQN, the second trained model based algorithm processor 9 relies on information observed in the previous N steps (N configurable, common choice is 10'000). First, a training batch needs to be constructed. Thereby, the second trained model based algorithm processor 9 loads information from randomly sampled previous steps, i.e. it loads their current states STA, taken updates UPD, observed reward REW, and next states STA. This step includes the construction of these states STA as described previously. Based on the sampled current states STA, the quality values QV are estimated for all possible actions using the described DNN. The same is done for the corresponding next states STA using a different DNN (referred to as the "target model"), which gets updated with the weights from the first DNN every M-th step (can be configured, usually set to $M = 10$). This procedure is common for DQN, as it is supposed to increase the stability of the algorithm. Now, the quality values QV for the update actions actually taken get updated according to the following formula, where $r_i$ is the reward in the i-th step, $v_{model}$ and $v_{target\_model}$ the output of the corresponding models, $y$ the depreciation factor, $\alpha$ is the learning rate, and $S_i / S_{i+1}$ the current / next state STA:

$$q_{i,new} = q_{i,current} + \alpha * (r_i + \gamma * \max_{a}\{v_{target_{model}}(S_{i+1})[a])\} - q_{i,current})$$

$$\rightarrow v_{model}(S_i)[a_i] = q_{i,new}$$

The model (first DNN) is then trained ('fitted') using with this constructed batch.

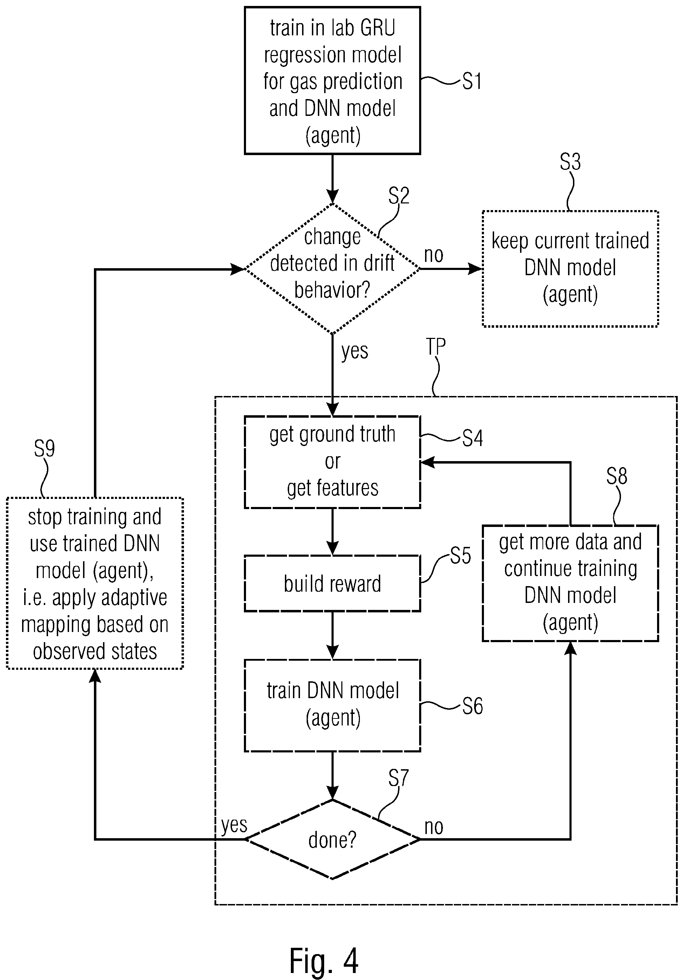

Figure 4 illustrates an exemplary method for operating the second or third exemplary embodiment of a gas sensing device 1 according to the disclosure.

According to embodiments of the disclosure, the update processor 8 is configured to detect a change of a drift behavior of one or more of the one or more chemo-resistive gas sensors 2, which can not be compensated by the updates UPD.

According to embodiments of the disclosure, the update processor 8 is configured to initiate a training procedure TP for training the second trained model in case that the change of the drift behavior, which can not be compensated by the updates UPD, is detected.

Figure 4 illustrates a typical implementation of the invention. In a step S1 a GRU regression network estimating the gas concentrations and the DNN need to be trained in the lab. The gas sensing device 1 can then be operated on a sensor board, its sensing results SR being continuously monitored / checked whether one of the chemo-resistive gas sensors 2 changes its drift behavior in a step S2. As long as no change of the drift behavior is detected, the operational mode is maintained in step S3.

As soon as a certain drift-level is detected or a change in drift behavior is observed, the DNN model needs to be trained and/or updated. In one implementation, the sensor is taken offline and the reference sensing results RSR are gathered (by for example consulting an analyzer, other sensors in operation, internet connection to a monitoring station, etc.) in step S4. The sensing results SR and the gathered reference sensing results RSR are now used to build a ground-truth aided reward REW in step S5 and to train a DNN model (RL agent) in step S6 as detailed above and illustrated in Figure 2. In step S7, a check is performed whether the training was successful. Further training may be executed based on further data gathered in step S8.

The implementation described above is based on the true gas concentrations and can therefore be operated only when the 'ground truth' is available. If this restriction can be dropped, the disclosed approach becomes even more powerful, as it would explore the full self-adaptive nature of reinforcement learning.

A second implementation here is to compare feature values FV that are affected in a different way by the drift (e.g. time and frequency domain features in case of baseline drift) and build in step S5 a features based reward REW as detailed above and illustrated in Figure 3.

After the training of the DNN model has finished, the trained second model based algorithm processor 9 can be used online on the gas sensing device 1 to correct the preprocessed signal samples PSS by gradually adapting the assumed underlying drift parameters (depending on the taken assumption about the drift) in step S9. The DNN now can be used as in Figure 1. Therefore, this proposed implementation can be viewed as a sophisticated way of learning the drift.

Figure 5 illustrates exemplary sensing results SR over time of a gas sensing device 1 according to the disclosure and exemplary prior art sensing results PSR over time of a gas sensing device according to prior art compared to reference sensing results RSR.

Figure 6 illustrates an exemplary features ratio $F_{nd}(t)/F_d(t)$ over time for different time intervals, wherein $F_d(t)$ is a drifting feature value FV of the sets of feature values FV and $F_{nd}(t)$ is a non-drifting feature value FV of the sets of feature values FV.

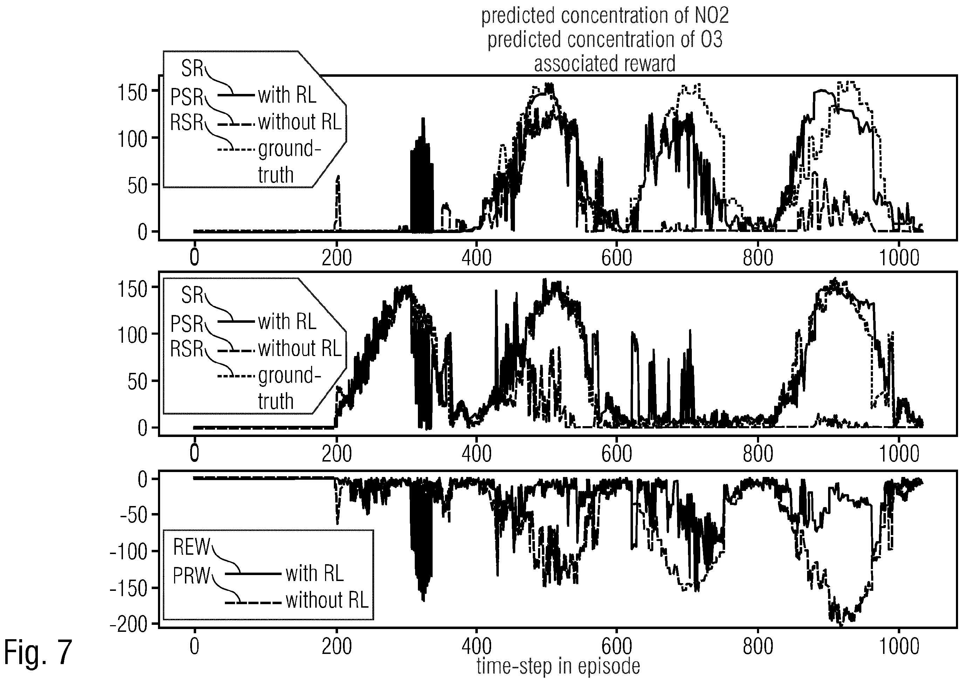

Figure 7 illustrates exemplary sensing results SR of a gas sensing device 1 according to the disclosure and exemplary prior art sensing results PSR of a gas sensing device according to prior art compared to reference sensing results RSR for nitrogen dioxide and ozone as well as the associated reward REW of the gas sensing device 1 according to the disclosure and the associated prior art reward PRW of the gas sensing device according to prior art.

The described approach has been tested on upward drifting multigas sensor data. The second trained model based algorithm processor 9 has been trained with 550 episodes with various drifts (positive as well as negative). For these experiments, the drift was mostly linear and equal for all of four chemo-resistive gas sensors 2 used in the experiments.

In Figure 7, the upper two subplots show nicely how the proposed approach is able to improve the estimation of the gas concentrations. The used gas sensor data starts to drift after 300 minutes. One can see that the estimation without RL almost instantly starts to fail, i.e. the sensing results SR simply drops to zero for both gases. With RL, the estimation continues to work till the end of the episode. This is also reflected in the last subplot showing the reward REW for these two cases. Over all, the gas sensing device 1 implementing the proposed drift correction technique achieves a much higher reward REW.

It can be seen how the device 1 is able to almost perfectly align the sensing results SR to the reference sensing results RSL.

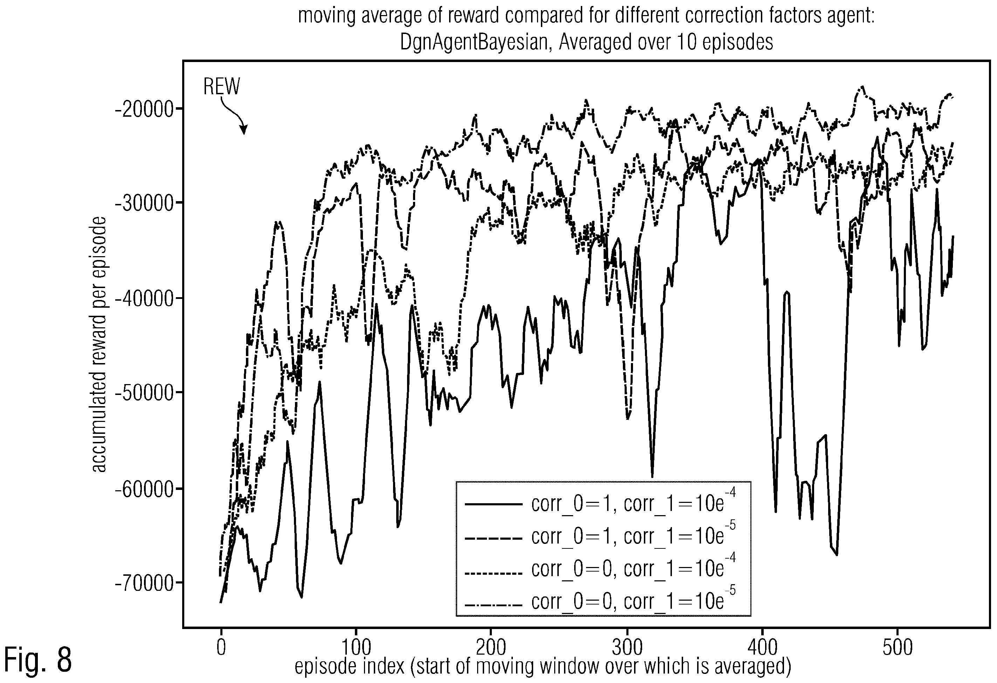

Figure 8 illustrates an exemplary accumulated reward REW for different correction factors over time.

Figure 8 shows the accumulated reward REW per episode for different correction factors of $a_0$ and $a_1$. One can see that the performance is influenced by this choice, for example using a factor of $10^{-5}$ for $a_1$ seems to work better than $10^{-4}$. Generally, this factor is a design choice needed to be made carefully based on the severity of the drift.

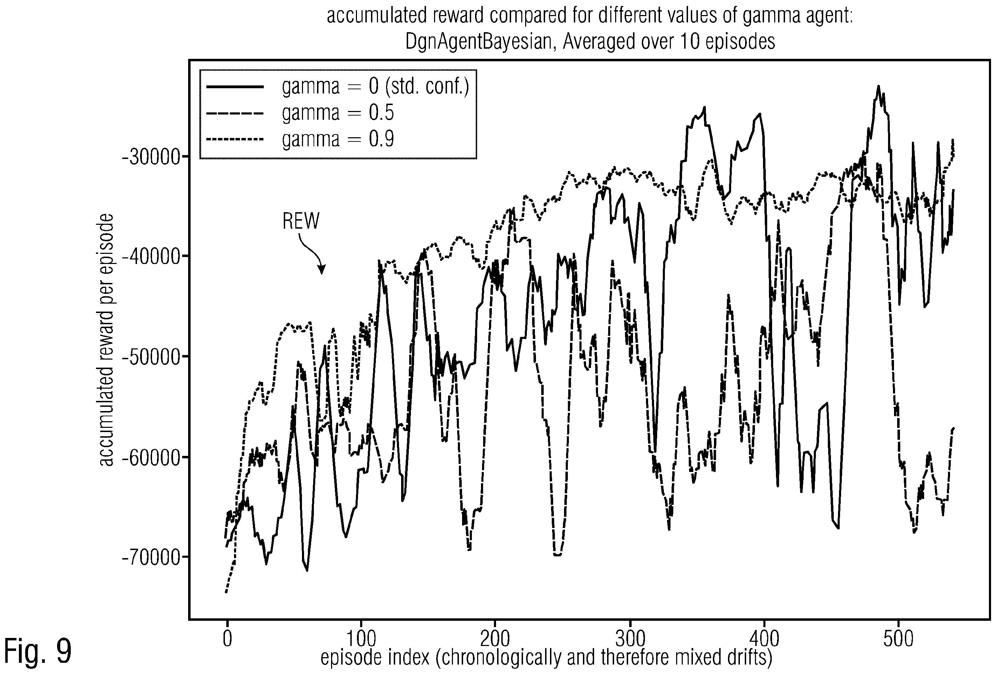

Figure 9 illustrates an exemplary accumulated reward REW for different depreciation factors over time.

Another configuration choice one can make is gamma ($\gamma$, can be viewed as the *depreciation factor* in DQN). A plot of the accumulated reward per episode is shown in Figure 9. We first notice that the agent is able to learn something useful (i.e. improve the reward REW over time) for all presented cases. Furthermore, the second trained model based algorithm processor 9 having the highest gamma (y = 0.9 in green) seems to be the most stable one. This is intuitive as a higher gamma generally weights steps lying further back more. Therefore, a second trained model based algorithm processor 9 with a high gamma is able to "look further into the future", which enables it to recover from bad states making the yielded accumulated reward REW per episode more consistent.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

The above described is merely illustrative, and it is understood that modifications and variations of the arrangements and the details described herein will be apparent to others skilled in the art. It is the intent, therefore, to be limited only by the scope of the impending claims and not by the specific details presented by way of description and explanation above.

References:

[1] Zhi-Pu Wang and Huai-Ning Wu. Gas Source Localization using Improved Multi-Agent Reinforcement Learning, 2020.

[2] Hoda S. Abdel-Aty-Zohdy and Mahmoud Al-Nsour. Reinforcement Learning Neural Network Circuits for Electronic Nose, 1999.

**Claims**

1. A gas sensing device for sensing one or more gases in a mixture of gases; the gas sensing device (1) comprising:

   one or more chemo-resistive gas sensors (2), wherein each of the gas sensors (2) is configured for generating signal samples (SIG) corresponding to concentrations of the one or more gases in the mixture of gases;
   a preprocessing processor (3) configured for preprocessing the signal samples (SIG) in order to generate a preprocessed signal sample (PSS) for each of the signal samples (SIG);
   a drift correction processor (4) configured for applying a mapping function (MPF) to each of the preprocessed signal samples (PSS) in order to create a drift-corrected signal sample (DCS) for each of the preprocessed signal samples (PSS), wherein the drift correction processor (4) is configured for receiving updates (UPD) for the mapping function (MPF), wherein each update (UPD) initiates an update step of a plurality of consecutive update steps;
   a feature extraction processor (5) configured for extracting a set of feature values (FV) from each of the drift-corrected signal samples (DCS) based on characteristics of the received drift-corrected signal samples (DCS);
   a sensing result processor (6) configured for creating for each of the gases a sensing result (SR) for each of the sets of feature values (FV), wherein the sensing result processor (6) comprises a first trained model based algorithm processor and a first trained model for the first trained model based algorithm processor, wherein the sets of feature values (FV) are fed to an input side of the first trained model based algorithm processor, and wherein the sensing results (SR) are provided at an output side of the first trained model based algorithm processor;
   a state processor (7) configured for determining a state (STA) of the gas sensing device (1) for a current update step of the update steps, wherein the state (STA) comprises at least a part of the preprocessed signal samples (PSS) of the current update step, at least a part of the preprocessed signal samples (PSS) of previous update steps of the update steps and the mapping function (MPF) of the current update step; and
   an update processor (8) configured for providing the update (UPD) for a subsequent update step subsequently following the current update step to the drift correction processor (4), wherein the update processor (8) comprises a second trained model based algorithm processor (9), a second trained model for the second trained model based algorithm processor (9) and an update action processor (10), wherein the state (STA) of the current update step is fed to an input side of the second trained model based algorithm processor (9), wherein a quality value (QV) for each possible update action of a plurality of update actions is provided at an output side of the second trained model based algorithm processor (9), wherein the update action processor (10) is configured for calculating the update (UPD) for the subsequent update step by applying the possible update action, which has a highest quality value (QV) of the quality values (QV) of the current update step, to the mapping function (MPF) of the current update step.

2. The gas sensing device according to the preceding claim, wherein the update processor (8) is configured to detect a change of a drift behavior of one or more of the one or more chemo-resistive gas sensors (2), which can not be compensated by the updates (UPD).

3. The gas sensing device according to the preceding claim, wherein the update processor (8) is configured to initiate a training procedure (TP) for training the second trained model in case that the change of the drift behavior, which can not be compensated by the updates (UPD), is detected.

4. The gas sensing device according to the preceding claim, wherein the second trained model based algorithm processor (9) is a reinforcement learning processor and the second trained model is a reinforcement learning model, wherein the gas sensing device (1) comprises a reward processor (11) configured for calculating a reward (REW), which is fed to an input side of the reinforcement learning processor (9) during the training procedure (TP), in order to train the reinforcement learning model.

5. The gas sensing device according to the preceding claim, wherein the reward processor (11) is configured for calculating the reward (REW) depending on the sensor results (SR) created by the sensing result processor (6) during the (TP) procedure and depending on reference sensing results (RSR) provided by an external source for the training procedure (TP).

6. The gas sensing device according to claim 4, wherein the reward processor (11) is configured for calculating the reward (REW) depending on the sets of feature values (FV) created by the feature extraction processor (5) during the training procedure (TP) by analyzing a first subset of the feature values (FV) of each set of feature values (FV) and by analyzing a second subset of the feature values (FV) of each set of feature values (FV), wherein the feature values (FV) of the first subset are more drift-dependent than the feature values (FV) of the second subset.

7. The gas sensing device according to one of the preceding claims, wherein the first trained model based algorithm processor is implemented as a gated recurrent unit regressor.

8. The gas sensing device according to one of the preceding claims, wherein the second trained model based algorithm processor (9) is implemented as a deep neural network.

9. A method for operating gas a sensing device (1) for sensing one or more gases in a mixture of gases, wherein the gas sensing device (1) comprises one or more chemo-resistive gas sensors (2), wherein the method comprises the steps of:

using each of the gas sensors (2) for generating signal samples (SIG) corresponding to concentrations of the one or more gases in the mixture of gases;
using a preprocessing processor (3) of the gas sensing device (1) for preprocessing the signal samples (SIG) in order to generate a preprocessed signal sample (PSS) for each of the signal samples (SIG);
using a drift correction processor (4) of the gas sensing device (1) for applying a mapping function (MPF) to each of the preprocessed signal samples (PSS) in order to create a drift-corrected signal sample (DCS) for each of the preprocessed signal samples (PSS), wherein the drift correction processor (4) is configured for receiving updates (UPD) for the mapping function (MPF), wherein each update (UPD) initiates an update step of a plurality of consecutive update steps;
using a feature extraction processor (5) of the gas sensing device (1) for extracting a set of feature values (FV) from each of the drift-corrected signal samples (DCS) based on characteristics of the received drift-corrected signal samples (DCS);
using a sensing result processor (6) of the gas sensing device (1) for creating for each of the gases a sensing result

(SR) for each of the sets of feature values (FV), wherein the sensing result processor (6) comprises a first trained model based algorithm processor and a first trained model for the first trained model based algorithm processor, wherein the sets of feature values (FV) are fed to an input side of the first trained model based algorithm processor, and wherein the sensing results (SR) are provided at an output side of the first trained model based algorithm processor;

using a state processor (7) of the gas sensing device (1) for determining a state (STA) of the gas sensing device (1) for a current update step of the update steps, wherein the state (STA) comprises at least a part of the preprocessed signal samples (PSS) of the current update step, at least a part of the preprocessed signal samples (PSS) of previous update steps of the update steps and the mapping function (MPF) of the current update step; and

using an update processor (8) of the gas sensing device (1) for providing the update (UPD) for a subsequent update step subsequently following the current update step to the drift correction processor (4), wherein the update processor (8) comprises a second trained model based algorithm processor (9), a second trained model for the second trained model based algorithm processor (9) and an update action processor (10), wherein the state (STA) of the current update step is fed to an input side of the second trained model based algorithm processor (9), wherein a quality value (QV) for each possible update action of a plurality of update actions is provided at an output side of the second trained model based algorithm processor (9), wherein the update action processor (10) is configured for calculating the update (UPD) for the subsequent update step by applying the possible update action, which has a highest quality value (QV) of the quality values of the current update step, to the mapping function (MPF) of the current update step.

**Patentansprüche**

1. Eine Gaserfassungsvorrichtung zum Erfassen eines oder mehrerer Gase in einem Gasgemisch; wobei die Gaserfassungsvorrichtung (1) folgende Merkmale aufweist:

einen oder mehrere chemoresistive Gassensoren (2), wobei jeder der Gassensoren (2) konfiguriert ist zum Erzeugen von Signalproben (SIG), die Konzentrationen des einen oder der mehreren Gase in dem Gasgemisch entsprechen;

einen Vorverarbeitungsprozessor (3), der konfiguriert ist zum Vorverarbeiten der Signalproben (SIG), um eine vorverarbeitete Signalprobe (PSS) für jede der Signalproben (SIG) zu erzeugen;

einen Driftkorrekturprozessor (4), der konfiguriert ist zum Anwenden einer Abbildungsfunktion (MPF) auf jede der vorverarbeiteten Signalproben (PSS), um eine driftkorrigierte Signalprobe (DCS) für jede der vorverarbeiteten Signalproben (PSS) zu erzeugen, wobei der Driftkorrekturprozessor (4) konfiguriert ist zum Empfangen von Aktualisierungen (UPD) für die Abbildungsfunktion (MPF), wobei jede Aktualisierung (UPD) einen Aktualisierungsschritt einer Mehrzahl von aufeinanderfolgenden Aktualisierungsschritten initiiert;

einen Merkmalsextraktionsprozessor (5), der konfiguriert ist zum Extrahieren eines Satzes von Merkmalswerten (FV) aus jeder der driftkorrigierten Signalproben (DCS) basierend auf Charakteristika der empfangenen driftkorrigierten Signalproben (DCS);

einen Erfassungsergebnisprozessor (6), der konfiguriert ist zum Erzeugen, für jedes der Gase, eines Erfassungsergebnisses (SR) für jeden der Sätze von Merkmalswerten (FV), wobei der Erfassungsergebnisprozessor (6) einen Erstes-Trainiertes-Modell-basierten Algorithmusprozessor und ein erstes trainiertes Modell für den Erstes-Trainiertes-Modell-basierten Algorithmusprozessor aufweist, wobei die Sätze von Merkmalswerten (FV) einer Eingangsseite des Erstes-Trainiertes-Modell-basierten-Algorithmusprozessors zugeführt werden, und wobei die Erfassungsergebnisse (SR) an einer Ausgangsseite des Erstes-Trainiertes-Modell-basierten Algorithmusprozessors bereitgestellt werden;

einen Zustandsprozessor (7), der konfiguriert ist zum Bestimmen eines Zustands (STA) der Gaserfassungsvorrichtung (1) für einen aktuellen Aktualisierungsschritt der Aktualisierungsschritte, wobei der Zustand (STA) zumindest einen Teil der vorverarbeiteten Signalproben (PSS) des aktuellen Aktualisierungsschritts, zumindest einen Teil der vorverarbeiteten Signalproben (PSS) von vorherigen Aktualisierungsschritten der Aktualisierungsschritte und die Abbildungsfunktion (MPF) des aktuellen Aktualisierungsschritts aufweist; und

einen Aktualisierungsprozessor (8), der konfiguriert ist zum Bereitstellen der Aktualisierung (UPD) für einen nachfolgenden Aktualisierungsschritt, der nachfolgend auf den aktuellen Aktualisierungsschritt folgt, an den Driftkorrekturprozessor (4), wobei der Aktualisierungsprozessor (8) einen Zweites-Trainiertes-Modell-basierten Algorithmusprozessor (9), ein zweites trainiertes Modell für den Zweites-Trainiertes-Modell-basierten Algorithmusprozessor (9) und einen Aktualisierungsaktionsprozessor (10) aufweist, wobei der Zustand (STA) des aktuellen Aktualisierungsschritts einer Eingangsseite des Zweites-Trainiertes-Modell-basierten Algorithmusprozessors (9) zugeführt wird, wobei ein Qualitätswert (QV) für jede mögliche Aktualisierungsaktion einer Mehrzahl von Aktualisierungsaktionen an einer Ausgangsseite des Zweites-Trainiertes-Modell-basierten Algorithmusprozessors (9) bereitgestellt wird, wobei der Aktualisierungsaktionsprozessor (10) konfiguriert ist zum Berechnen der Aktualisierung (UPD) für den nachfolgenden Aktualisierungsschritt durch Anwenden der möglichen Aktualisierungsaktion, die einen höchsten Qualitätswert (QV) der Qualitätswerte (QV) des aktuellen Aktualisierungsschritts aufweist, auf die Abbildungsfunktion (MPF) des aktuellen Aktualisierungsschritts.

2. Die Gaserfassungsvorrichtung gemäß dem vorhergehenden Anspruch, wobei der Aktualisierungsprozessor (8) konfiguriert ist zum Detektieren einer Änderung eines Driftverhaltens von einem oder mehreren des einen oder der mehreren chemoresistiven Gassensoren (2), die durch die Aktualisierungen (UPD) nicht kompensiert werden können.

3. Die Gaserfassungsvorrichtung gemäß dem vorhergehenden Anspruch, wobei der Aktualisierungsprozessor (8) konfiguriert ist zum Initiieren einer Trainingsprozedur (TP) zum Trainieren des zweiten trainierten Modells, falls die Änderung des Driftverhaltens, die durch die Aktualisierungen (UPD) nicht kompensiert werden kann, detektiert wird.

4. Die Gaserfassungsvorrichtung gemäß dem vorhergehenden Anspruch, wobei der Zweites-Trainiertes-Modell-basierte Algorithmusprozessor (9) ein Verstärkungslernprozessor ist und das zweite trainierte Modell ein Verstärkungslernmodell ist, wobei die Gaserfassungsvorrichtung (1) einen Belohnungsprozessor (11) aufweist, der konfiguriert ist zum Berechnen einer Belohnung (REW), die einer Eingangsseite des Verstärkungslernprozessors (9) während der Trainingsprozedur (TP) zugeführt wird, um das Verstärkungslernmodell zu trainieren.

5. Die Gaserfassungsvorrichtung gemäß dem vorhergehenden Anspruch, wobei der Belohnungsprozessor (11) konfiguriert ist zum Berechnen der Belohnung (REW) in Abhängigkeit von den Sensorergebnissen (SR), die durch den Erfassungsergebnisprozessor (6) während der (TP) Prozedur erzeugt werden, und in Abhängigkeit von Referenzerfassungsergebnissen (RSR), die durch eine externe Quelle für die Trainingsprozedur (TP) bereitgestellt werden.

6. Die Gaserfassungsvorrichtung gemäß Anspruch 4, wobei der Belohnungsprozessor (11) konfiguriert ist zum Berechnen der Belohnung (REW) in Abhängigkeit von den Sätzen von Merkmalswerten (FV), die durch den Merkmalsextraktionsprozessor (5) während der Trainingsprozedur (TP) erzeugt werden, durch Analysieren einer ersten Teilmenge der Merkmalswerte (FV) von jedem Satz von Merkmalswerten (FV) und durch Analysieren einer zweiten Teilmenge der Merkmalswerte (FV) von jedem Satz von Merkmalswerten (FV), wobei die Merkmalswerte (FV) der ersten Teilmenge driftabhängiger sind als die Merkmalswerte (FV) der zweiten Teilmenge.

7. Die Gaserfassungsvorrichtung gemäß einem der vorhergehenden Ansprüche, wobei der Erstes-Trainiertes-Modell-basierte Algorithmusprozessor als ein Gated-Recurrent-Unit-Regressor implementiert ist.

8. Die Gaserfassungsvorrichtung gemäß einem der vorhergehenden Ansprüche, wobei der Zweites-Trainiertes-Modell-basierte Algorithmusprozessor (9) als ein tiefes neuronales Netzwerk implementiert ist.

9. Ein Verfahren zum Betreiben einer Gaserfassungsvorrichtung (1) zum Erfassen eines oder mehrerer Gase in einem Gasgemisch, wobei die Gaserfassungsvorrichtung (1) einen oder mehrere chemoresistive Gassensoren (2) aufweist, wobei das Verfahren folgende Schritte aufweist:

Verwenden jedes der Gassensoren (2) zum Erzeugen von Signalproben (SIG), die Konzentrationen des einen oder der mehreren Gase in dem Gasgemisch entsprechen;
Verwenden eines Vorverarbeitungsprozessors (3) der Gaserfassungsvorrichtung (1) zum Vorverarbeiten der Signalproben (SIG), um eine vorverarbeitete Signalprobe (PSS) für jede der Signalproben (SIG) zu erzeugen;
Verwenden eines Driftkorrekturprozessors (4) der Gaserfassungsvorrichtung (1) zum Anwenden einer Abbildungsfunktion (MPF) auf jede der vorverarbeiteten Signalproben (PSS), um eine driftkorrigierte Signalprobe (DCS) für jede der vorverarbeiteten Signalproben (PSS) zu erzeugen, wobei der Driftkorrekturprozessor (4) konfiguriert ist zum Empfangen von Aktualisierungen (UPD) für die Abbildungsfunktion (MPF), wobei jede Aktualisierung (UPD) einen Aktualisierungsschritt einer Mehrzahl von aufeinanderfolgenden Aktualisierungsschritten initiiert;
Verwenden eines Merkmalsextraktionsprozessors (5) der Gaserfassungsvorrichtung (1) zum Extrahieren eines Satzes von Merkmalswerten (FV) aus jeder der driftkorrigierten Signalproben (DCS) basierend auf Charakteristika der empfangenen driftkorrigierten Signalproben (DCS);

Verwenden eines Erfassungsergebnisprozessors (6) der Gaserfassungsvorrichtung (1) zum Erzeugen, für jedes der Gase, eines Erfassungsergebnisses (SR) für jeden der Sätze von Merkmalswerten (FV), wobei der Erfassungsergebnisprozessor (6) einen Erstes-Trainiertes-Modell-basierten Algorithmusprozessor und ein erstes trainiertes Modell für den Erstes-Trainiertes-Modell-basierten Algorithmusprozessor aufweist, wobei die Sätze von Merkmalswerten (FV) einer Eingangsseite des Erstes-Trainiertes-Modell-basierten Algorithmusprozessors zugeführt werden, und wobei die Erfassungsergebnisse (SR) an einer Ausgangsseite des Erstes-Trainiertes-Modell-basierten Algorithmusprozessors bereitgestellt werden;

Verwenden eines Zustandsprozessors (7) der Gaserfassungsvorrichtung (1) zum Bestimmen eines Zustands (STA) der Gaserfassungsvorrichtung (1) für einen aktuellen Aktualisierungsschritt der Aktualisierungsschritte, wobei der Zustand (STA) zumindest einen Teil der vorverarbeiteten Signalproben (PSS) des aktuellen Aktualisierungsschritts, zumindest einen Teil der vorverarbeiteten Signalproben (PSS) von vorherigen Aktualisierungsschritten der Aktualisierungsschritte und die Abbildungsfunktion (MPF) des aktuellen Aktualisierungsschritts aufweist; und

Verwenden eines Aktualisierungsprozessors (8) der Gaserfassungsvorrichtung (1) zum Bereitstellen der Aktualisierung (UPD) für einen nachfolgenden Aktualisierungsschritt, der nachfolgend auf den aktuellen Aktualisierungsschritt folgt, an den Driftkorrekturprozessor (4), wobei der Aktualisierungsprozessor (8) einen Zweites-Trainiertes-Modell-basierten Algorithmusprozessor (9), ein zweites trainiertes Modell für den Zweites-Trainiertes-Modell-basierten Algorithmusprozessor (9) und einen Aktualisierungsaktionsprozessor (10) aufweist, wobei der Zustand (STA) des aktuellen Aktualisierungsschritts einer Eingangsseite des Zweites-Trainiertes-Modell-basierten Algorithmusprozessors (9) zugeführt wird, wobei ein Qualitätswert (QV) für jede mögliche Aktualisierungsaktion einer Mehrzahl von Aktualisierungsaktionen an einer Ausgangsseite des Zweites-Trainiertes-Modell-basierten Algorithmusprozessors (9) bereitgestellt wird, wobei der Aktualisierungsaktionsprozessor (10) konfiguriert ist zum Berechnen der Aktualisierung (UPD) für den nachfolgenden Aktualisierungsschritt durch Anwenden der möglichen Aktualisierungsaktion, die einen höchsten Qualitätswert (QV) der Qualitätswerte des aktuellen Aktualisierungsschritts aufweist, auf die Abbildungsfunktion (MPF) des aktuellen Aktualisierungsschritts.

## Revendications

1. Un dispositif de détection de gaz pour détecter un ou plusieurs gaz dans un mélange de gaz ; le dispositif (1) de détection de gaz comprenant :

un ou plusieurs capteurs (2) de gaz chemio-résistants, dans lequel chacun des capteurs (2) de gaz est configuré pour créer des échantillons (SIG) de signal correspondant à des concentrations du un ou des plusieurs gaz dans le mélange de gaz ;

un processeur (3) de traitement préalable configuré pour traiter préalablement les échantillons (SIG) de signal afin de créer un échantillon (PSS) de signal traité au préalable pour chacun des échantillons (SIG) de signal ;

un processeur (4) de correction de dérive configuré pour appliquer une fonction (MPF) de correspondance à chacun des échantillons (PSS) de signal traités au préalable afin de créer un échantillon (DCS) de signal corrigé en dérive pour chacun des échantillons (PSS) de signal traités au préalable, dans lequel le processeur (4) de correction de dérive est configuré pour recevoir des mises à jour (UPD) de la fonction (MPF) de correspondance, dans lequel chaque mise à jour (UPD) initie un stade de mise à jour d'une pluralité de stades consécutifs de mise à jour ;

un processeur (5) d'extraction de caractéristiques configuré pour extraire un ensemble de valeurs (FV) de caractéristiques de chacun des échantillons (DCS) de signal corrigé en dérive sur la base des caractéristiques des échantillons (DCS) de signal corrigé en dérive reçus ;

un processeur (6) de résultat de détection configuré pour créer pour chacun des gaz un résultat (SR) de détection pour chacun des ensembles de valeurs (FV) de caractéristiques, dans lequel le processeur (6) de résultat de détection comprend un processeur d'algorithme reposant sur un premier modèle entraîné et un premier modèle entraîné pour le processeur d'algorithme reposant sur le premier modèle entraîné, dans lequel l'ensemble de valeurs (FV) de caractéristiques sont envoyées à un côté d'entrée du processeur d'algorithme reposant sur le premier modèle entraîné, et dans lequel les résultats (SR) de détection sont donnés à un côté de sortie du processeur d'algorithme reposant sur le premier modèle entraîné ;

un processeur (7) d'état configuré pour déterminer un état (STA) du dispositif (1) de détection de gaz pour un stade en cours de mise à jour des stades de mise à jour, dans lequel l'état (STA) comprend au moins une partie des échantillons (PSS) de signal traités au préalable du stade en cours de mise à jour, au moins une partie des échantillons (PSS) de signal traités au préalable de stades précédents de mise à jour des stades de mise à jour et la fonction (MPF) de correspondance du stade en cours de mise à jour ; et

un processeur (8) de mise à jour configuré pour donner la mise à jour (UPD) pour un stade subséquent de mise à jour suivant ensuite le stade en cours de mise à jour au processeur (4) de correction de dérive, dans lequel le processeur (8) de mise à jour comprend un processeur (9) d'algorithme reposant sur un deuxième modèle entraîné, un deuxième modèle entraîné pour le processeur (9) d'algorithme reposant sur un deuxième modèle entraîné et un processeur (10) d'action de mise à jour, dans lequel l'état (STA) du stade en cours de mise à jour est envoyé à un côté d'entrée du processeur (9) d'algorithme reposant sur un deuxième modèle entraîné, dans lequel une valeur (QV) de qualité pour chaque action possible de mise à jour d'une pluralité d'actions de mise à jour est donnée à un côté de sortie du processeur (9) d'algorithme reposant sur un deuxième modèle entraîné, dans lequel le processeur (10) d'action de mise à jour est configuré pour calculer la mise à jour (UPD) pour le stade subséquent suivant de mise à jour en appliquant l'action possible de mise à jour, qui a la valeur (QV) de qualité la plus haute des valeurs (QV) de qualité du stade en cours de mise à jour, à la fonction (MPF) de correspondance du stade en cours de mise à jour.

2. Le dispositif de détection de gaz suivant la revendication précédente, dans lequel le processeur (8) de mise à jour est configuré pour détecter un changement de comportement de dérive du un ou des plusieurs du un ou des plusieurs capteurs (2) de gaz chemio-résistants, qui ne peut pas être compensé par les mises à jour (UPD).

3. Le dispositif de détection de gaz suivant la revendication précédente, dans lequel le processeur (8) de mise à jour est configuré pour initier une procédure (TP) d'entraînement pour entraîner le deuxième modèle entraîné dans le cas où le changement du comportement de dérive, qui ne peut pas être compensé par les mises à jour (UPD), est détecté.

4. Le dispositif de détection de gaz suivant la revendication précédente, dans lequel le processeur (9) d'algorithme reposant sur un deuxième modèle entraîné est un procédé d'enseignement de renfort et le deuxième modèle entraîné est un modèle d'enseignement de renfort, dans lequel le dispositif (1) de détection de gaz comprend un processeur (11) de récompense configuré pour calculer une récompense (REW), qui est envoyée à un côté d'entrée du processeur (9) d'enseignement de renfort pendant la procédure (TP) d'entraînement, afin d'entraîner le modèle d'enseignement de renfort.

5. Le dispositif de détection de gaz suivant la revendication précédente, dans lequel le processeur (11) de récompense est configuré pour calculer la récompense (REW) en fonction des résultats (SR) de capteur créés par le processeur (6) de résultat de détection pendant la procédure (TP) et en fonction des résultats (RSR) de détection de référence donnés par une source extérieure pour la procédure (TP) d'entraînement.

6. Le dispositif de détection de gaz suivant la revendication 4, dans lequel le processeur (11) de récompense est configuré pour calculer la récompense (REW) en fonction des ensembles de valeurs (FV) de caractéristiques créés par le processeur (5) d'extraction de caractéristiques pendant la procédure (TP) d'entraînement en analysant un premier sous-ensemble des valeurs (FV) de caractéristiques de chaque ensemble de valeurs (FV) de caractéristiques et en analysant un deuxième sous-ensemble de valeurs (FV) de caractéristiques de chaque ensemble de valeurs (FV) de caractéristiques, dans lequel les valeurs (FV) de caractéristiques du premier sous-ensemble dépendent plus de la dérive que les valeurs (FV) de caractéristiques du deuxième sous-ensemble.

7. Le dispositif de détection de gaz suivant l'une des revendications précédentes, dans lequel le processeur d'algorithme reposant sur un premier modèle entraîné est mis en œuvre sous la forme d'un régresseur à unité récurrente fermée.

8. Le dispositif de détection de gaz suivant l'une des revendications précédentes, dans lequel le processeur (9) d'algorithme reposant sur un deuxième modèle entraîné est mis en œuvre sous la forme d'un réseau neuronal profond.

9. Un procédé pour faire fonctionner un dispositif (1) de détection de gaz pour détecter un ou plusieurs gaz dans un mélange de gaz, dans lequel le dispositif (1) de détection de gaz comprend un ou plusieurs capteurs (2) de gaz chemio-résistants, dans lequel le procédé comprend les stades de :

utiliser chacun des capteurs (2) de gaz pour créer des échantillons (SIG) de signal correspondant à des concentrations du ou des plusieurs gaz dans le mélange de gaz ;

utiliser un processeur (3) de traitement préalable du dispositif (1) de détection de gaz pour traiter de manière préalable les échantillons (SIG) de signal afin de créer un échantillon (PSS) de signal traité au préalable pour chacun des échantillons (SIG) de signal ;

utiliser un processeur (4) de correction de dérive du dispositif (1) de détection de gaz pour appliquer une fonction (MPF) de correspondance à chacun des échantillons (PSS) de signal traités au préalable, afin de créer un échantillon (DCS) de signal corrigé en dérive pour chacun des échantillons (PSS) de signal traités au préalable, dans lequel le processeur (4) de correction de dérive est configuré pour recevoir des mises à jour (UPD) de la fonction (MPF) de correspondance, dans lequel chaque mise à jour (UPD) initie un stade de mise à jour d'une pluralité de stades consécutifs de mise à jour ;

utiliser un processeur (5) d'extraction de caractéristiques du dispositif (1) de détection de gaz pour extraire un ensemble de valeurs (FV) de caractéristiques de chacun des échantillons (DCS) de signal corrigé en dérive sur la base de caractéristiques des échantillons (DCS) de signal corrigé en dérive ;

utiliser un processeur (6) de résultat de détection du dispositif (1) de détection de gaz pour créer pour chacun des gaz un résultat (SR) de détection pour chacun des ensembles de valeurs (FV) de caractéristiques, dans lequel le processeur (6) de résultat de détection comprend un processeur d'algorithme reposant sur un premier modèle entraîné et un premier modèle entraîné pour le processeur d'algorithme reposant sur le premier modèle entraîné, dans lequel l'ensemble de valeurs (FV) de caractéristiques sont envoyées à un côté d'entrée du processeur d'algorithme reposant sur le premier modèle entraîné, et dans lequel les résultats (SR) de détection sont donnés à un côté de sortie du processeur d'algorithme reposant sur le premier modèle entraîné ;

utiliser un processeur (7) d'état du dispositif (1) de détection de gaz pour déterminer un état (STA) du dispositif (1) de détection de gaz pour un stade en cours de mise à jour des stades de mise à jour, dans lequel l'état (STA) comprend au moins une partie des échantillons (PSS) de signal traités au préalable du stade en cours de mise à jour, au moins une partie des échantillons (PSS) de signal traités au préalable de stades précédents de mise à jour des stades de mise à jour et la fonction (MPF) de correspondance du stade en cours de mise à jour ; et

utiliser un processeur (8) de mise à jour du dispositif (1) de détection de gaz pour donner la mise à jour (UPD) pour un stade subséquent de mise à jour suivant ensuite le stade en cours de mise à jour au processeur (4) de correction de dérive, dans lequel le processeur (8) de mise à jour comprend un processeur (9) d'algorithme reposant sur un deuxième modèle entraîné, un deuxième modèle entraîné pour le processeur (9) d'algorithme reposant sur un deuxième modèle entraîné et un processeur (10) d'action de mise à jour, dans lequel l'état (STA) du stade en cours de mise à jour est envoyé à un côté d'entrée du processeur (9) d'algorithme reposant sur un deuxième modèle entraîné, dans lequel une valeur (QV) de qualité pour chaque action possible de mise à jour d'une pluralité d'actions de mise à jour est donnée à un côté de sortie du processeur (9) d'algorithme reposant sur un deuxième modèle entraîné, dans lequel le processeur (10) d'action de mise à jour est configuré pour calculer la mise à jour (UPD) pour le stade subséquent suivant de mise à jour en appliquant l'action possible de mise à jour, qui a la valeur (QV) de qualité la plus haute des valeurs (QV) de qualité du stade en cours de mise à jour à la fonction (MPF) de correspondance du stade en cours de mise à jour.

Fig. 1

EP 4 414 699 B1

Fig. 2

EP 4 414 699 B1

Fig. 3

EP 4 414 699 B1

Fig. 4

sensing results for heavy drift

Fig. 5

EP 4 414 699 B1

Fig. 6

Fig. 7

EP 4 414 699 B1

moving average of reward compared for different correction factors agent:
DgnAgentBayesian, Averaged over 10 episodes

Fig. 8

accumulated reward compared for different values of gamma agent:
DgnAgentBayesian, Averaged over 10 episodes

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3936861 A

**Non-patent literature cited in the description**

- **ZHI-PU WANG ; HUAI-NING WU**. *Gas Source Localization using Improved Multi-Agent Reinforcement Learning*, 2020

- **HODA S ; ABDEL-ATY-ZOHDY ; MAHMOUD AL-NSOUR**. *Reinforcement Learning Neural Network Circuits for Electronic Nose*, 1999